# EUROPEAN PATENT APPLICATION

(11) **EP 4 282 963 A1**
(43) Date of publication of application: **29.11.2023**
(21) Application number: 22174870.0
(22) Date of filing: 23.05.2022
(51) Int. Cl.: C12N 15/11, C12N 15/113, A61K 31/7088

(54) **NUCLEIC ACID MODIFIED BIOLOGICAL CELL WITH EXPANSION-DEPENDENT GENE EXPRESSION**

(71) Applicant: Eberhard Karls Universität Tübingen, Medizinische Fakultät, 72074 Tübingen (DE)
(72) Inventor: Haraszti, Reka, 72074 Tübingen (DE); Schaible, Philipp, 70825 Münchingen (DE); Ryaykenen, Tatyana, 70191 Stuttgart (DE); Kremer, Anastasia, 72074 Tübingen (DE)
(74) Representative: Witte, Weller & Partner Patentanwälte mbB

(57) **Abstract**

The present invention relates to a nucleic acid modified biological cell and a method for modulating, in a nucleic acid modified cell, the expression of a nucleotide sequence encoding a target.

## Description

The present invention relates to a nucleic acid modified biological cell and a method for modulating, in a nucleic acid modified cell, the expression of a nucleotide sequence encoding a target.

### FIELD OF THE INVENTION

The present invention generally relates to the field of cell or molecular biology, especially to the field of cell therapy. More particular, it relates to a therapeutically effective biological cell produced by means of genetic engineering.

### BACKGROUND OF THE INVENTION

Cell therapy uses viable cells which are injected, grafted or implanted into a patient in order to effectuate a medicinal effect. For example, T cells are transplanted which are capable of fighting cancer cells via cell-mediated immunity in the course of immunotherapy, or stem cells are grafted to regenerate diseased tissues.

Chimeric antigen receptors (CAR) bearing cells are central tools of cell therapeutic approaches. CAR enable engineered T cells to eliminate cells displaying antigens targeted via the CAR. Autologous CAR T cells are successfully deployed to treat B cell malignancies, with four approved products targeting CD19, and one targeting the B cell maturation antigen. Several other CAR T cell products are under development with more than a hundred ongoing clinical trials for hematologic malignancies and solid tumors. CAR T cells may also be employed against autoimmune diseases and viral infections.

Despite initial success of CAR T cells, several challenges continue to hamper their widespread clinical use. Primary resistance occurs in 10-20 % and relapse is also common with an incidence of 30-50%. Resistance mechanisms within the infused T cells include failure to expand *in vivo*, early exhaustion, poor persistence, insufficient fitness etc. The tumor itself may also contribute to resistance in form of target antigen loss, downregulation or mutation. CAR T cell mediated toxicities in form of cytokine release syndrome - CRS, or immune effector cell associated neurotoxicity syndrome - ICANS vary from mild to severe and lead to an 1.5% fatality rate. Long-lasting and/or severe neutropenia as well as B cell aplasia may lead to fatal infections.

Currently approved CAR T cells represent a heterogeneous population of clones with varying efficacy. This heterogeneity is attributed to (1) lentiviral vector based manufacturing leading to incidental genomic integration, and (2) phenotypic heterogeneity of the apheresis product.

Efficacious CAR T cell clones are able to proliferate, eliminate targeted cells, do not get exhausted, and persist *in vivo* for several months up to one year. However, hyperactive CAR T cell clones - with too much proliferation and no exhaustion cause toxicities.

Several T cell modulation strategies are being tested to reduce T cell mediated resistance and toxicity as well as to expand the circle of targetable diseases. These modulation strategies are based on the current understanding of why certain CAR T cell clones are more efficacious than others.

One of such modulation strategies involve the use of so-called small interfering RNA (siRNA). Using siRNA in cell therapies to modulate the cells' activity is, e.g., disclosed in WO2015/084897. Alternatively, so-called small hairpin RNA (shRNA) are employed in cell therapies for this purpose as, e.g., disclosed in WO2020/206248, WO2019/138354, WO2015/136001, or WO2011/059836.

However, the problem of cell therapy insufficient effectivity and, in particular, toxicities has not been satisfactorily addressed in the state of the art.

Against this background, it is an object of the present invention to provide a biological cell that can be used in the context of a cell therapy without causing the problems, especially toxicities, described for current cell therapies. In particular, it is intended to provide such a biological cell that allows temporal control of the expression of a target gene or protein so that expression occurs either at the beginning of an expansion phase or at a late expansion phase.

### SUMMARY OF THE INVENTION

This object underlying the invention is met by the provision of a nucleic acid modified biological cell comprising
- comprised by its genome
   (i) a nucleic acid comprising a first expressible nucleotide sequence encoding a target,
   (ii) a recombinant nucleic acid comprising a second expressible nucleotide sequence configured to modulate the expression and/or function of said first nucleotide sequence, and
- non-comprised by its genome a nucleic acid comprising a third nucleotide sequence configured to inhibit the expression and/or function of said second nucleotide sequence.

This object is also met by a method for modulating, in a nucleic acid modified cell, the expression of a first nucleotide sequence encoding a target, comprising:
1) provision of a biological cell comprising, integrated in the genome of biological cell, a first expressible nucleotide sequence encoding a target protein,
2) integrating into the genome of said biological cell a recombinant nucleic acid comprising a second expressible nucleotide sequence configured to modulate the expression and/or function of said first nucleotide sequence,
3) introducing into said biological cell outside of its genome a nucleic acid comprising a third nucleotide sequence configured to inhibit the expression and/or function of said second nucleotide sequence.

According to the invention, a "biological cell" includes eukaryotic cells, such as animal (e.g. mammalian or human cells) and plant cells, and prokaryotic cells (e.g. bacterial cells).

"Nucleic acid modified" means, according to the invention, that the cell and/or its nucleic acid, e.g. DNA or RNA, is modified by genetic engineering techniques, such as methods of nucleic acid recombination, but, e.g., also by introducing, into the cell, artificial non-recombining nucleic acid molecules. It can, therefore, synonymously also referred to a "genetically modified" biological cell.

The biological cell according to the invention has a genome, i.e. genetic information, typically in form of DNA, that is heritable. All genetic information that is part of the genome of the biological cell is copied in the course of proliferation or cell division and transferred to the progeny cells. Consequently, the genome and the genetic information contained therein are not lost through proliferation, i.e. they are 'proliferation-independent'. The genome of a cell from higher, e.g. eukaryotic organisms, is typically formed by chromosomes. Extrachromosomal elements, such as episomes, can be introduced by biotechnological means and likewise form part of the cell's genome.

According to the invention, a "nucleotide sequence" is the sequence of nucleotides of a nucleic acid, deoxyribonucleic acid (DNA) or ribonucleic acid (RNA). It may consist of natural and/or chemically modified nucleotides.

According to the invention the genome of the biological cell comprises a first nucleotide sequence that is expressible and encodes a target, e.g. a target protein or a non-translated target RNA or non-protein target, respectively. In a preferred embodiment, the first protein is encoded by a gene represented by the first expressible nucleotide sequence.

According to the invention the genome of the biological cell comprises a second nucleotide sequence that has been made part of or is integrated into the genome of the cell. Said integration can be realized by methods of nucleic acid recombination or genetic engineering, respectively. In a preferred embodiment said second nucleotide sequence has been integrated into a cell's chromosome or was introduced into the cell on an extrachromosomal element, such as an episome.

"Expressible" means that the first and second nucleotide sequence is readable by cellular structures, preferably mediated by an upstream promoter, and convertible to a nucleic acid transcript, such as an mRNA, and, in a preferred embodiment, translatable into a target protein.

The cell according to the invention, further comprises a third nucleotide sequence that is not part of the genome. It is introduced into the cell by methods well known to the skilled artisan, such as conjugate-mediated delivery, electroporation or lipid nanoparticles. Because the third nucleotide sequence is not part of or integrated into the genome, the third nucleotide sequence is not copied in the course of cell proliferation and is not passed on to the progeny cells. The third nucleotide sequence is therefore "diluted" by cell division events in the progeny cells and is lost in the course of proliferation. The third nucleotide sequence is therefore 'proliferation-dependent'.

The third nucleotide sequence has a structure that is capable of inhibiting the expression of the second nucleotide sequence, e.g., in an embodiment, by steric interaction between the third and second sequences (e.g., by annealing via complementary sequence segments) or enzymatically chaptalized interaction, preventing the latter from being read and expressed.

The inventors have recognized that in the modified biological cell according to the invention, the expression of the first nucleotide sequence or target can be controlled in such a way that it is expressed over time either at the beginning of a proliferation or expansion phase (e.g. at the beginning of cell therapy) or later in or at the end of the proliferation/expansion phase. The first and second nucleotide sequences, as part of the genome, get duplicated during mitosis and copy numbers remain unchanged in daughter cells. Their expressions remain insensitive to cell proliferation. The third nucleotide sequence is present in the biological cell at a certain concentration and gets diluted with each cell division. The duration of effect can be set by adjusting initial concentrations used. The combination of the second and third nucleotide sequences with opposing effects are introduced into the cell. As the third nucleotide sequence dilutes out in the course of proliferation the second nucleotide sequence will get released from its blockage and start exerting its effect, e.g. expressing a protein or downregulating the target.

The cell according to the invention thus creates optimal conditions for use in cell therapy. By exploiting the dilution effect acting on the third nucleotide sequence, it is possible to achieve that the target, e.g. a specific cellular gene, is exclusively expressed in the cell according to the invention at the beginning of a therapeutic expansion burst, before activity-induced proliferation, and is inhibited thereafter, or is exclusively expressed at the end of a therapeutic expansion burst, after activity-induced proliferation, and is inhibited prior thereto. This timely-controlled and/or limited expression of the target significantly reduces the toxicities and side effects frequently observed in the state of the art of cell therapies, such as cytokine release syndrome in CAR-T cells, or graft-versus-host disease in donor lymphocyte infusions. It may also enhance therapeutically relevant proliferation in safe manner, or enrich for a therapeutically beneficial phenotype at specific time widows of an expansion burst, thereby enhancing efficacy of the cell therapy.

The biological cell according to the invention allows to use a cell therapeutic window that efficacious, non-toxic cell clones reach and persist within, never exceeding its upper limit. The inventors have found that the timing of a modulation strategy is crucial to ensure maintenance of this therapeutic window. In an embodiment of the invention, proliferation and tissue penetration should be accelerated and exhaustion inhibited early on to enable reaching the therapeutic window. Proliferation should be slowed down at a later phase to flatten the curve and prevent over-activation. Thus, cell modulation strategy should ideally be sensitive to the expansion phase. The invention satisfies these and other needs.

In an embodiment of the invention said second expressible nucleotide sequence is configured to inhibit the expression of said first nucleotide sequence.

This measure creates the constructive conditions for strong expression of the target to be possible at the beginning of the proliferation phase and for this expression of the target to be inhibited during the course of proliferation. Thus, the second nucleotide sequence is itself inhibited at the beginning by the third nucleotide sequence. After a few replication cycles, the third nucleotide sequence is progressively diluted out, losing its inhibitory effect on the second nucleotide sequence. The second nucleotide sequence can then increasingly exert its inhibitory effect on the target, so that the target can be completely inhibited after a few replication cycles and cell divisions.

In another embodiment of the biological cell according to the invention said second expressible nucleotide sequence is integrated into a chromosome and/or an episome of said biological cell.

This measure ensures that the second nucleotide sequence is or becomes part of the genome of the biological cell and is passed on to progeny cells with each cell division. An episome is a plasmid that forms an extrachromosomal element, which remains part of the eukaryotic genome without integration. Episomes manage this by replicating together with the rest of the genome and subsequently associating with metaphase chromosomes during mitosis. Episomes do not degrade unlike standard plasmids and are not epigenetically silenced inside eukaryotic cell nucleus.

In yet another embodiment of the invention said nucleic acid comprising the third nucleotide sequence is an oligonucleotide.

This measure creates the constructive conditions for the temporary presence of the third nucleotide sequence in the cell. In this context, an oligonucleotide is understood to be an oligomer constructed from a few nucleotides (DNA or RNA), typically between 7 and 30 nucleotides, sometimes 7-10 nucleotides. It is understood that the specific number of nucleotides may vary, depending on the application. Further, the oligonucleotide may comprise natural nucleotides and/or chemically modified nucleotides. In this context, additional influence can be exerted on the stability of the oligonucleotide and thus on the duration of dilution or "thinning" via the type of chemical modification, in particular when using RNA. Typically, stability is regulated by the modification of uridine and cytidine nucleotides. Examples for chemically modified uridine include pseudouridine (Ψ), 2-thiouridine, 5-methyluridine, 5-idouridine, 4-thiouridine, 5-bromouridine, 2'-methyl-2'-deoxyuridine, 2'-amino-2'-deoxyuridine, 2'-azido-2'-deoxyuridine, 2'-fluoro-2'-deoxyuridine. Examples for chemically modified cytidine include 5-methylcytidine (m5C), 3-methylcytidine, 2-thiocytidine, 2'-methyl-2'-deoxcytidin, 2'-amino-2'-deoxycytidine, 2'-fluoro-2'-deoxycytidine, 5-iodcytidine, 5-bromocytidine, and 2'-azido-2'-deoxycytidine. Chemical modifications also include such affecting the sugars and the phosphate backbone, such as 2'-fluoro, 2'-O-methyl, 2-methoxyethyl, LNA, phosphorothioate, vinylphosphonate and many more.

In another embodiment of the invention said second expressible nucleotide sequence and said third nucleotide sequence encode a regulatory RNA.

This measure advantageously realizes the type of oligonucleotide that can be particularly well and easily designed as an inhibitor of the expression of the second nucleotide sequence. Regulatory RNA is a group of RNA molecules which do not encode protein but can perform a multitude of cellular functions, including the inhibition of the expression and/or function of another nucleotide sequence.

In yet another embodiment of the invention said second expressible nucleotide sequence encode an RNA selected from the group consisting of: shRNA (small/short hairpin RNA), snRNA (small nuclear RNA), miRNA (microRNA), miRNA sponge, piRNA, IncRNA (long non-coding RNA), guide RNA, mRNA (messenger RNA), antisense transcript, transposon.

These measures advantageously provide the second expressible nucleotide sequence in a form such that it can be integrated into the genome of the biological cell and, when expressed, modulate the expression of said first nucleotide sequence, e.g. via RNA interference.

In a further embodiment of the invention said third nucleotide sequence encode an RNA selected from the group consisting of: siRNA (small interfering RNA), ASO (antisense oligonucleotide), miRNA (microRNA), AMO (anti-miRNA), aptamer.

This measure has the advantage that such oligonucleotides comprising the third nucleotide sequence are provided which allow an effective inhibition of the expression of the second nucleotide sequence, e.g. via hybridization, RNA interference, RNA silencing and similar mechanisms.

In another preferred embodiment in step (3) of the method according to the invention said nucleic acid comprising the third nucleotide sequence is introduced into said biological cell via a method selected from the group consisting of: electroporation, lipid conjugation delivery, aptamer conjugation delivery, lipid nanoparticle delivery, viral vector delivery, antibody conjugation delivery, small molecule conjugation delivery, peptide conjugation delivery, extracellular vesicle delivery.

This measure has the advantage of using proven and effective methods in the state of the art for introducing oligonucleotides into biological cells.

In still another embodiment in step (2) of the method according to the invention said recombinant nucleic acid comprising the second expressible nucleotide sequence is introduced into said biological cell via an integrating and/or episomal virus, thereby adding the recombinant nucleic acid as part of the genome.

This embodiment employs well-established methods of recombination biology that allow safe and reliable integration of the second nucleotide sequence into the genome of the biological cell.

In another embodiment of the invention said biological cell is an immune cell.

With this further development, such cells are nucleic acid modified according to the invention, which are particularly suitable as a tool for cell therapy.

In still another embodiment of the invention said biological or immune cell is selected from the group consisting of: T cell, CAR T cell, tumor infiltrating lymphocyte, donor lymphocyte infusion, NK cell, CAR NK cell, B cell, dendritic cell, macrophage, tumor infiltrating macrophage, hematopoietic stem cell, including allogenic afore-listed cells.

The above cells are particularly suitable and effective for use in cell therapy. As used herein, the term "chimeric antigen receptor" or "CAR" refers to an engineered receptor that confers or grafts specificity for an antigen onto an immune effector cell (e.g., a human T cell). A chimeric antigen receptor comprises at least an extracellular ligand-binding domain or moiety, a transmembrane domain, and an intracellular domain that comprises one or more signaling domains and/or co-stimulatory domains. A "CAR T cell", therefore, refer to a T cell that has been genetically engineered to produce said artificial T cell receptor. The receptors are chimeric because they combine both antigen-binding and T cell activating functions into a single receptor.

T cells can be genetically modified to express a CAR construct that enables them to recognize and target surface markers on so-called "cancer stem cells". In the prior art, this often results in the problem that these surface markers are not exclusively expressed on cancer cells, whereby these "traditional" CAR T cells also recognize and target non-tumor cells. This means that targeting "cancer stem cells" comes with severe side effects. These side effects severely limit the use of these CAR T cells. According to the findings of the inventors, these CAR T cells can be genetically modified to express another CAR construct in addition to the described first CAR construct, since CAR T cells can express multiple CAR constructs. This second CAR construct can target a gene which recognizes as specific as possible a protein that is exclusively expressed on cancer cells. The CAR T cells can use this CAR construct to find the tumor. This is where the invention comes in. The "first" CAR construct used by the CAR T cells to recognize the so-called "cancer stem cells" is down-regulated using oligonucleotides with the third nucleotide sequence at the beginning of the expansion phase. Thus, the CAR T cells recognize only tumor cells and side effects are prevented. Once the CAR T cells have recognized the tumor, by means of the "second" CAR construct, which is permanently expressed, and expand, the oligonucleotides dilute. As a result, the CAR T cells of the invention, which are located in the tumor and therefore expand, express the CAR construct with which they can recognize and target the "cancer stem cells". The invention thus makes it possible to combat "cancer stem cells" with few side effects.

In another embodiment of the invention said first expressible nucleotide sequence encoding the target protein is a gene selected from the group consisting of: BRD4, p300, TET2, DNMT3, miR-15/16, miR-150, GLUT1, TOX, Blimp-1, NR4A, BATF, IRF4, NFAT, KLRG1, EOMES, TBX2, PRDM1, GZMA, HZMB, PRF1, IFNG, PD1, TIM3, LAG3, TIGIT, CTLA4, miR-146a, miR-155, MHC-I, MHC-II, miR-17-92, CX3CR1, HLA-E, HLA-G, Siglec7/9.

This measure has the advantage of addressing such target genes that should preferably be downregulated in an early expansion phase of the cells, especially CAR T cells.

In yet another embodiment of the invention said first expressible nucleotide sequence encoding the target protein is a gene selected from the group consisting of: IL-1, IL-6. GM-CSFm CD40L, LAG3, CD69, TCF1, BCL-6, BMI1, FOXO1, KLF2, LEF1, TCF7, IL2RA, CD27, TNF, CCR7, SELL, CD62L, miR-143, CCR5, CCR2, let7, TGFBeta, IL-12, IL-18, IL-23, T cell receptor, miR-27a, IL-7, CCL19, CCL21, CAR constructs targeting the tumor microenvironment, IL-15, IL-7, CAR construct targeting a cancer stem cell marker.

This measure has the advantage that, on the other hand, such target genes are addressed that should preferably be downregulated in a late expansion phase of the cells, especially the CAR T cells.

Another subject-matter of the invention relates to the nucleic acid modified biological cell of any of the preceding claims for use in medicine, preferably for use in the treatment and/or prophylaxis of a disease selected from the group consisting of: cancer, autoimmune disease, alloimmunity (rejection or graft versus host disease), viral infection, bacterial infection.

The features, characteristics and advantages disclosed for the cell or the modulating method according to the invention apply likewise to the use according to the invention.

Still another subject-matter of the invention relates to method for the treatment of a living being in need by administering the nucleic acid modified biological cell according to the invention.

The features, characteristics and advantages disclosed for the cell or the modulating method according to the invention apply likewise to the treatment method according to the invention.

The "living being", according to the invention, relates to any animal or vegetable being, especially a mammal, including a human being.

In an embodiment of the treatment method according to the invention said living being is suffering from a disease selected from the group consisting of: cancer, autoimmunity, viral infection.

This measure has the advantage that the invention can be used specifically for the treatment of those diseases for which cell therapy is particularly suitable.

It is to be understood that the before-mentioned features and those to be mentioned in the following cannot only be used in the combination indicated in the respective case, but also in other combinations or in an isolated manner without departing from the scope of the invention.

The invention is now described and explained in further detail by referring to the following non-limiting examples and drawings.

### BRIEF DESCRIPTION OF THE DRAWINGS

- Figure 1: CAR T cell expansion modulation in specific time windows.
- Figure 2: Proliferation sensitive inhibition - early.
- Figure 3: Proliferation sensitive inhibition - delayed.
- Figure 4: Silencing duration is dose dependent in rapidly dividing T lymphoblasts.
- Figure 5: Silencing last at least 13 days in activated T cells.
- Figure 6: Increasing inhibition of Luciferase transgene in Jurkat T lymphoblasts.

### EXAMPLES

### 1. General

### CAR T cells - Need for Speed-Up

Chimeric antigen receptors (CAR) enable engineered T cells to eliminate cells displaying antigens targeted via the CAR. Autologous CAR T cells are successfully deployed to treat B cell malignancies, with four approved products targeting CD19, and one targeting the B cell maturation antigen. Several other CAR T cell products are under development with more than a hundred ongoing clinical trials for hematologic malignancies and solid tumors. CAR T cells may also be employed against autoimmune diseases and viral infections.

Despite initial success of CAR T cells, several challenges continue to hamper their widespread clinical use. Primary resistance occurs in 10-20 % and relapse is also common with an incidence of 30-50%. Resistance mechanisms within the infused T cells include failure to expand *in vivo,* early exhaustion, poor persistence, insufficient fitness etc. The tumor itself may also contribute to resistance in form of target antigen loss, downregulation or mutation. CAR T cell mediated toxicities in form of cytokine release syndrome - CRS, or immune effector cell associated neurotoxicity syndrome - ICANS vary from mild to severe and lead to an 1.5% fatality rate. Long-lasting and/or severe neutropenia as well as B cell aplasia may lead to fatal infections.

Several T cell modulation strategies are being tested to reduce T cell mediated resistance and toxicity as well as to expand the circle of targetable diseases. These modulation strategies are based on the current understanding of why certain CAR T cell clones are more efficacious than others.

Currently approved CAR T cells represent a heterogeneous population of clones with varying efficacy. This heterogeneity is attributed to (1) lentiviral vector based manufacturing leading to incidental genomic integration11, and (2) phenotypic heterogeneity of the apheresis product.

Precise genomic insertion of the CAR construct via CRISPR or temporary CAR expression via mRNAs circumvents the lentivirus induced incidental integration problem.

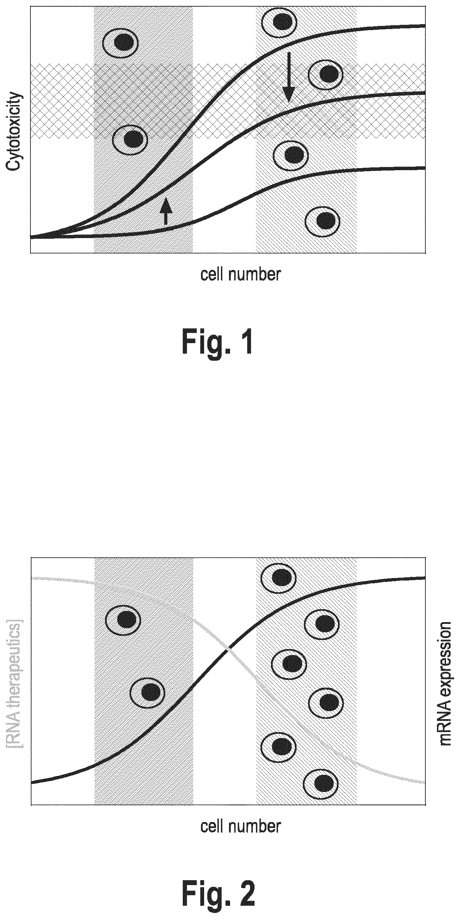

Efficacious CAR T cell clones are able to proliferate, eliminate targeted cells, do not get exhausted, and persist in vivo for several months up to one year. However, hyperactive CAR T cell clones - with too much proliferation and no exhaustion cause toxicities. Thus, the inventors propose a therapeutic window that efficacious, non-toxic CAR T cell clones reach and persist within, never exceeding its upper limit (Figure 1.).

The timing of a modulation strategy is crucial to ensure maintenance of this therapeutic window. Proliferation and tissue penetration should be accelerated and exhaustion inhibited early on to enable reaching the therapeutic window. Proliferation should be slowed down at a later phase to flatten the curve and prevent over-activation (Figure 1). Thus, CAR T cell modulation strategy should ideally be sensitive to the expansion phase.

### RNA treated CAR T cells

Regulatory RNAs are able to program a phenotype via fine-tuning transcription, splicing, translation and mRNA lifespan. RNA therapeutics, therefore, can be used for rational phenotype design of i.e. CAR T cells.

RNA therapeutics encompass various reversible and titratable sequence-specific inhibitors. Inhibition may depend on cell expansion.

RNA interference directs short double stranded RNAs (miRNAs or siRNAs) against complementary mRNAs to induce mRNA degradation. Antisense oligonucleotides (ASOs) display diverse mechanisms of action to cleave pre-mRNAs and to modulate translation and splicing. Four approved siRNAs and three approved ASOs demonstrate the potency of RNA therapeutics for clinically relevant phenotype modulation.

RNA therapeutics are being explored to potentiate CAR T cells. Several studies have demonstrated feasibility and utility of RNA interference or ASO mediated silencing in primary T and CAR T cells. siRNAs can be efficiently delivered to T cells *ex vivo* using either electroporation, lipid-conjugated siRNAs, aptamer-conjugated siRNA, lipid nanoparticles or viral vectors (shRNA). Three clinical trials are currently testing the clinical utility of RNA-modified CAR T cells. The combination of shRNA against multiple targets may be of advantage when modulating a complex system such as a CAR T cell.

### Proliferation sensitive expression control

The inventors propose a combination of an RNA therapeutic and a genetic engineering strategy to achieve proliferation-sensitive silencing in CAR T cells.

RNA therapeutics (e.g. siRNAs, artificial miRNAs, ASOs and anti-miRs) are chemically synthesized oligonucleotides exogenously introduced to cells. However, genetic engineering can also be used to introduce the same therapeutic mechanisms to a cell. For example, siRNAs and miRNAs can be encoded into short hairpins (shRNA) and delivered via integrating or episomal viruses. Viral delivery may also introduce engineered small nucleolar RNAs targeting pre-mRNAs for degradation ref and miRNA sponges or decoys to antagonize miRNA function.

A major difference between RNA therapeutics and genetic engineering is the timeline: the effect of RNA therapeutics is temporary while the effect of genetic engineering is permanent.

The duration of effect of an RNA therapeutic depends on (i) its metabolic stability, and (ii) the cell proliferation rate. Extensive chemical modifications enhance the metabolic stability of RNA therapeutics and extend their duration of effect to 6 months in non-dividing hepatocytes of patients. However, the intracellular concentration of an RNA therapeutic gets halved with every cell division and the duration of effect in rapidly dividing cells remains largely unknown. In case of siRNAs 50 - 3.000 fold more siRNA can be delivered to cells than what is necessary for silencing - therefore a dilution effect is expected to appear after 5-12 cell divisions. Indeed, we observed a silencing duration of 4-30 divisions in primary immune cells and immune cell lines.

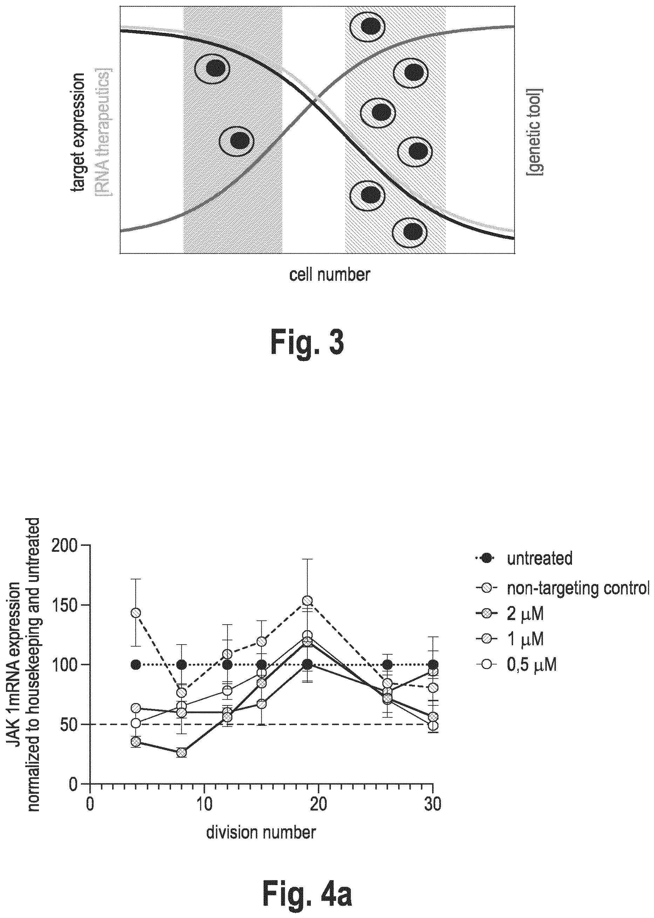

The combination of an RNA therapeutic and a genetic engineering strategy enables three sensitivity levels to cell proliferation:
(1) Proliferation insensitive: A genetic engineering tool (i.e. shRNA, snRNA, miRNA sponge) may be expressed from a sequence integrated into the genome or from an episome. Both chromosomes and extrachromosomal episomes get duplicated during mitosis and copy numbers remain unchanged in daughter cells. Therefore, the expression of the genetic engineering tool only depends on the promoter used and remain insensitive to cell proliferation.
(2) Proliferation sensitive inhibition: An RNA therapeutic (i.e. siRNA, artificial miRNA, ASO, anti-miR) enters the cell at a certain concentration and gets diluted with each cell division. The duration of effect can be set by adjusting initial concentrations used (Figure 2).
(3) Proliferation sensitive expression: The combination of an RNA therapeutic and a genetic engineering tool with opposing effects (i.e. anti-miR - shRNA, miRNA - miRNA sponge, siRNA - mRNA), are delivered to a cell. The RNA therapeutic blocks the effect of the genetic engineering tool. As the RNA therapeutic dilutes out, the genetic engineering tool will get released from this blockage and start exerting its effects - i.e. expressing a protein or downregulating its targets (Figure 3).

### Specific interventions in selected time-windows

### Toxicity control

The timing of a T cell modulation strategy may enable to differentiate between toxicity and anti-tumor activity - two otherwise overlapping mechanisms.

IL-1 and IL-6 are optimal targets for delayed proliferation sensitive inhibition. While IL-1/IL-6 inhibition is a common toxicity management strategy, it also impairs therapeutic activity of T cells. Delayed inhibition of IL-1/IL-6, however, allows chemokine production throughout the therapeutically necessary CAR T cell proliferation and only inhibits overaction. Delayed inhibition of the IL-1/IL-6 axis may be possible via (1) delayed expression of IL-1/IL-6 inhibitors, (2) delayed silencing of GM-CSF (responsible for humoral activation of IL-1/IL-6 producing macrophages), or (3) delayed silencing of CD40L54, CD6955, LAG356 (responsible of contact-dependent activation of macrophages).

Lower expression levels of the CAR construct or reduced CAR T cell number may lead to reduced toxicity but intact therapeutic activity. The CAR expression level may be controlled via a precise genetic integration site, or perhaps by pre-treatment or post-administration treatment with RNA therapeutics. Delayed silencing of the CAR construct may enable specific inhibition of over-activation.

Proliferation sensitive inhibition may be superior to genetically engineered on and off switches (e.g. lenalidomide-, asunaprevir- or rimiducid-gated switches successfully applied in CAR T cells in preclinical settings) for toxicity control, since ProsIT enables prevention while on/off switches only curtail toxicities that already occurred.

### Phenotype optimization

Successful T-cell therapies display a phenotype predestining them for robust antitumor activity and limited toxicity. RNA based modification may enable to initially enrich for this successful phenotype, while letting cells differentiate and exhaust in a delayed fashion to ensure anti-tumor effective function and a healthy lifetime. Lower degree of T cell differentiation, such as memory phenotype correlates with better anti-tumor outcomes. The scarcity of such cells makes it difficult to rely on apheresis as memory T cell supply for CAR T cell production. Therefore, several strategies have been pursued to induce memory phenotype including small molecule inhibitors and interleukins - concepts that have reached the clinical trial stage (NCT02652910, NCT01087294). However, some of these treatments have been found to limit the differentiation capacity of memory T cells into effector T cells, impairing anti-tumor effects. Therefore, after initial enrichment of a memory phenotype (*ex vivo* production), cells should be able to differentiate into an effector phenotype (in patients).

Memory T cells express TCF1, BCL-6, BMI1, FOXO1, KLF2, LEF1, TCF7, IL2RA, CD27, TNF, CCR7, SELL, CD62L. Initial exogenous expression of these genes may be able to reprogram cells into a memory phenotype. However, loss of expression (via delayed inhibition) should occur analogue to natural cell differentiation. Inhibition of BRD4 and p300, TET2, miR-15/16 or miR-150 led to memory phenotype and superior anti-tumor activity. Overexpression of miR-143 or downregulation of the miR-143 target GLUT1 via siRNA also promoted memory T cell formation. These interventions may be timed to the *ex vivo* production phase (early inhibition) in order to improve anti-tumor activity.

Effector T cell phenotype is characterized by expression of TOX, Blimp-1, NR4A, BATF, IRF4, NFAT, KLRG1, EOMES, TBX2, PRDM1, GZMA, HZMB, PRF1, IFNG and may express exhaustion markers PD1, TIM3, LAG3, TIGIT, CTLA4 and by miR 146a. Early inhibition of these genes may be able eliminate effector cells from the CAR T cell product and block early exhaustion. However, re-expression of effector genes should be allowed for natural cell differentiation and successful anti-tumor effect (early inhibition only). Persistence of effector T cells is promoted by miR-17-92, miR-155. These genes should ideally be expressed in a delayed fashion, in parallel with T cell differentiation into the effector phenotype.

It remains yet to be elucidated, whether above genes only correlate with or drive memory/effector phenotypes. Phenotype driver genes would be ideal targets for RNA-preprogramming of CAR T cells. Rather evidence-based, less mechanism-based target identification may emerge from RNAi or CRISPRi screens of CAR T cells as well as integration site analysis of approved CAR T cell products.

### CAR T Cell Enhancement in solid tumors

Precise expression timing of infiltration promoting, microenvironment fighting and antigen recognizing genes may potentiate the so far underwhelming activity of solid tumor targeting CAR T cells.

### Infiltration

Infiltration as well as proliferation could be enhanced by inhibiting inhibitory receptors on CAR T cells, such as PD1, TIM3 and LAG3 or CX3CR1. In fact, the combined silencing of these factors via shRNAs is concept that already entered the clinical trial phase. Overexpression of miRNAs targeting above receptors has also proved to be beneficial. Early inhibition of these factors may enhance safety via allowing a proliferation control in a delayed fashion.

CAR T cells engineered to co-express IL-7 and CCL19 or IL-7 and CCL21 - chemokines important to establish T cell zones in lymphoid organs - show better infiltration and higher activity against solid tumors than traditional CAR T cells (clinical trial NCT03778346). However, IL-7, CCL19 and CCL21 overexpression is potentially carcinogenic. Early inhibition of the above inhibitory factors may enhance safety via allowing a proliferation control in a delayed fashion.

Overexpression of receptors recognizing chemokines with high abundance in solid tumors (CCR2 62, CCR563) or inhibiting their inhibitors appear promising strategies to promote infiltration. However, CCR5 may mediate non-classical apoptosis pathway within the tumor, resulting in (CAR) T cell death. Hence, CCR5 is an ideal candidate for delayed silencing.

### Microenvironment

Several genetic engineering strategies exist to overcome the tumor microenvironment. Tumor vasculature may be targeted via CARs. CAR T cells may express matrix-degrading enzymes. Immunosuppressive solute receptors may be inhibited via either dominant-negative knock-in, shRNAs or CRISRP. Alternatively, immunosuppressive solutes can be targeted via CAR T cells. Immunosuppressive cell types, such as Tregs, tumor-associated macrophages, myeloid-derived suppressor cells or cancer-associated fibroblasts can also be targeted via CAR T cells.

Generally, tumor microenvironment degradation should ideally be a local reaction, thus, CAR T cells should ideally exert a tumor microenvironment inhibiting or degrading activity once docked on the tumor. Therefore, tumor microenvironment targeting should rather happen in a delayed fashion.

### Armed CAR T cells

CAR T cells may be "armed" to produce pro-inflammatory cytokines normally expressed by other cell types. CAR T cells expressing IL-12, IL-18, IL-15, IL-7, TGFBeta, and IL-23 achieve higher efficacy against solid tumors. However, constitutive expression of these cytokines may be severely toxic Therefore, cytokine production should only occur, once CAR T cells are docked on the tumor and proliferate locally. Early inhibition/delayed expression may achieve this goal, enhancing armed CAR T cell safety.

miR-155 overexpression has also been shown to enhance T cell activity against solid tumors. An artificial miRNA here may be able to prevent toxicities due to over-activation of T cells.

### Antigen recognition

Cancer stem cell targeting have been pursued as a strategy to prevent relapse and overcome tumor associated antigen heterogeneity. However, cancer stem cell markers are often not specific to tumors, leading to severe on-target off-tumor toxicities. CD133 - the most commonly used cancer stem cell marker - is also expressed in hematopoietic stem cells human embryonic stem cells and epithelial cells of several tissues. Delayed expression of a cancer stem cell specific CAR in combination with the early expression of a tumor associated marker specific CAR may increase efficacy and safety.

### Allogeneic CAR T cells

"Off the shelf" allogenic CAR T cells may be a game-changer in the field, shortening production length and improving initial T cell fitness. However, allogeneic T cells are often rejected via the host immune system or induce graft-versus-host disease.

A genetic engineering strategy to overcome allorejection include MHC I knock-out (β2-microglobulin locus). However, MHC deficient CAR T cells are vulnerable to NK cell mediated destruction. This NK-mediated "missing-self" reaction may be avoided by expressing NK-inhibiting receptors, such as HLA-E or HLA-G or Siglec7/9. Class II MHC molecules may also be knocked out by targeting CIITA. A promising approach is the targeting of the 4-1BB receptor on activated alloimmune lymphocytes via expression of a defense receptor on allogeneic CAR T cells. However, complete blockage of alloimmunity in allogeneic T cells eliminates an important proliferation control and may lead to over-activation. Therefore, allorejection eliminating strategies may be restricted to early proliferation phase to enhance safety.

T cell receptor deficiency may circumvent GvHD induced by allogeneic CAR T cells. One strategy is to knock out the constant α chain of the T cell receptor. Alternatively, shRNA mediated silencing of the TCR has been successfully used in adoptive T cell therapies. Another strategy is the exchange of the TCR for the CAR construct via CRISPR. However, the T cell receptor is thought to be involved in low level "tonic" signaling, important for T cell maintenance and effector function. Therefore, the T cell receptor is promising target for delayed silencing.

### 2. Material and methods

### siRNAs

The siRNAs were fully modified with 2'-fluoro, 2'-OMe and phosphorothioates as described previously. Lipid conjugates included cholesterol and divalent myristic acid, synthesis as described previously.

### Cell culture

Jurkat cells were passaged twice and week and cultured at 1 million cells/ml in RPMI supplemented with 10% FBS and 1%P/S.

NanoLuc expressing Jurkat cells were obtained from Christian Seitz, University Tübingen and cultured as described above.

### Generation of activated T cells

Buffy coats from health blood donors were obtained from the blood donation center at the University Hospital Tübingen. Peripheral blood mononuclear cells were obtained from buffy coats via Ficoll density centrifugation. 1.5 million cells are plated per 24-well in 1 ml of RPMI supplemented with 10% FBS, 1% P/S and 25 mM HEPES. MACSiBeads were added in a cell-to-bead ratio of 2:1 (Miltenyi human T cell Activation/Expansion kit). Cells were incubated with the beads for 3 days and beads were then removed vie centrifugation and activated cells plated at a density of 1.5 million/ml. Medium was now supplemented in addition with IL-7 and IL-15.

### siRNA treatment

siRNA was added to cells at a final concentration indicated on the Figures (0.5, 1, 2 or 4 µM). Cells were cultured as described above. At each passage cells were counted, cells were harvested for downstream mRNA quantification assay and the rest subcultured at a density described above. This procedure was repeated at each passage.

### mRNA quantification

QuantiGene Singleplex assay (Invitrogen) was used to quantify mRNAs, following manufacturer's protocols.

### Luminescence Measurement

Jurkat cells stably expressing Nanoluc were plated in 96 well plate at a density of 1 million cells/ml in phenolred-free RPMI supplemented with 10% FBS and 1% P/S. Cells were treated with various concentrations of siRNA targeting either NanoLuc or non-targeting control. On day 3 furimazine (ChemShuttle) was added to the cells at a concentration of 1µM and luminescence measured using a Tecan MPlex plate reader. Then cells were transferred back to the incubator. On day 7 the same procedure was repeated. Before returning to the incubator, cells were, however passaged and replated at a density of 1 million cells/ml. On following days implicated on the figures the procedure was repeated.

### 3. Results

In order to demonstrate proliferation sensitive inhibition, we treated Jurkat cells (human T lymphoblast cell line) and primary human T cells with siRNAs targeting PPIB and JAK1 as well as non-targeting control (NTC). Cells were passaged in the presence of siRNA and maintained in culture for 30 days. At each passage a sample was taken to quantify mRNA levels.

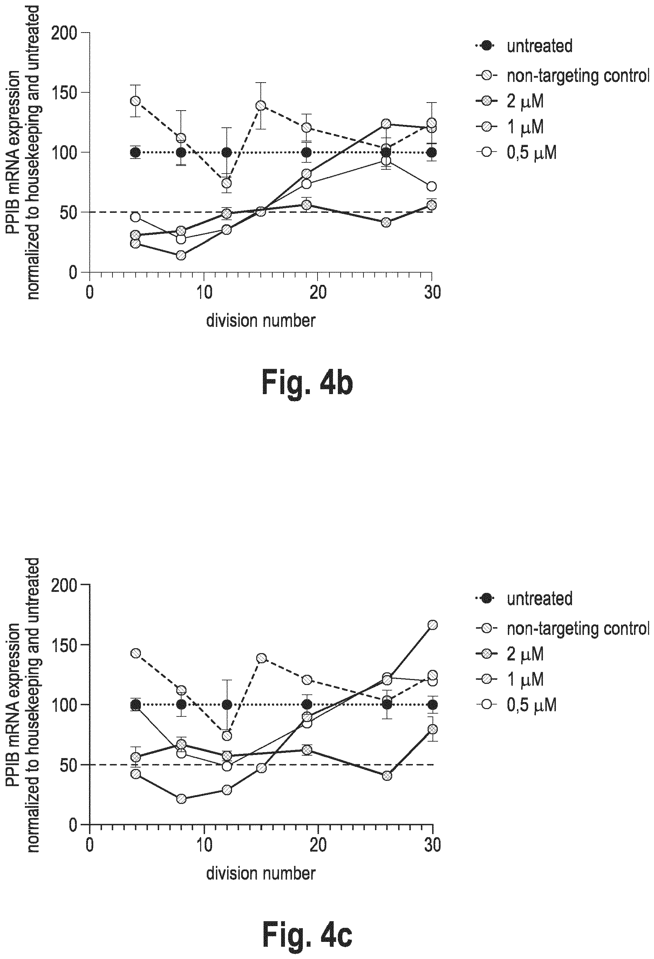

Jurkat T lymphoblasts were treated with various concentrations of cholesterol conjugated siRNA targeting JAK1, divalent myristic acid conjugated siRNA targeting PPIB or cholesterol conjugated siRNA targeting PPIB. Cells were passaged 1:3 twice a week. At each passage a sample was taken, cells lysed and mRNA expression analyzed using QuantiGene assay. Target mRNA expression was normalized to housekeeping gene (HPRT) and to untreated control. The outcome of this experiment can be seen in Figure 4. Depending on the target mRNA, the initial siRNA dose as well as the identity of the lipid conjugate on the siRNA the inventors have observed the duration of silencing (at least 50%) 14 to 30 days.

Proliferation sensitive inhibition could be shown in human primary T cells as well. Activated T cells generated from PBMCs were treated with siRNAs targeting PPIB or JAK1 and subcultured in the presence of IL-7 and IL-15. siRNA was not removed from the culture.

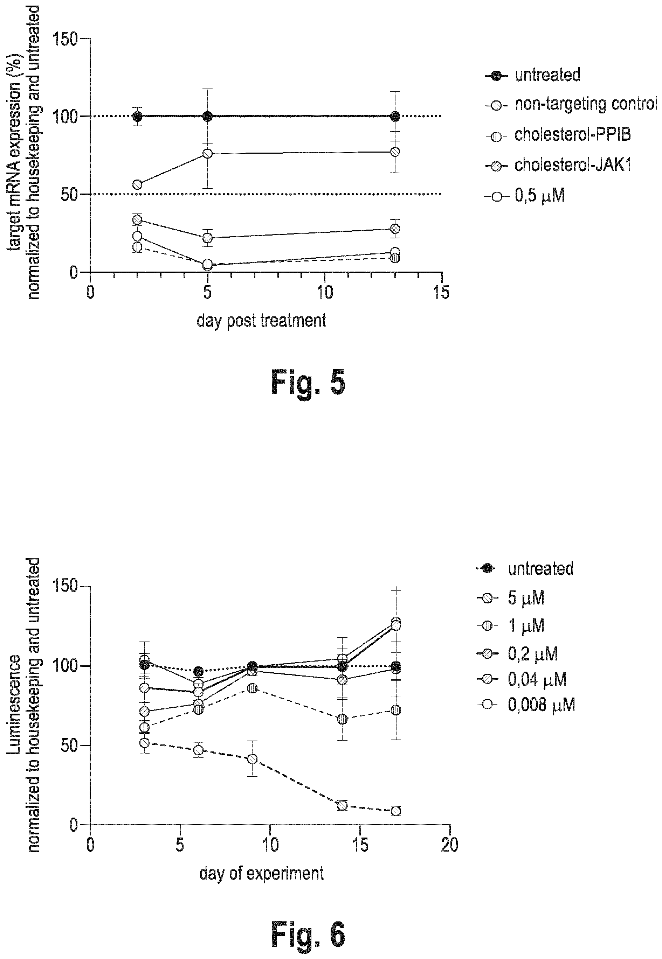

Activated T cells were treated with 2 µM of divalent myristic acid or cholesterol conjugated siRNA targeting either JAK1 or PPIB. Cells were passaged at each doubling. At each passage a sample was taken, cells lysed and mRNA expression analyzed using QuantiGene assay following manufacturer's protocols. Target mRNA expression (PPIB) was normalized to housekeeping gene (HPRT) and to untreated control. The result can be derived from Figure 5. The inventors observed a very efficient silencing that lasted for at least 13 days.

In order to demonstrate proliferation sensitive expression Jurkat T Lymphoblasts stably expressing NanoLuc transgene were treated with siRNA against NanoLuc at varying concentrations and luminescence measured twice a week.

Jurkat cells stably expressing NanoLuc were treated with cholesterol conjugated siRNA targeting NanoLuc, cells were passaged twice a week and luminescence measured at each passage. The outcome of this experiment is depicted in Figure 6. The inventors observed inhibition of the Luciferase that increased upon cell proliferation.

## Claims

1. A nucleic acid modified biological cell comprising
- comprised by its genome
(i) a nucleic acid comprising a first expressible nucleotide sequence encoding a target,
(ii) a recombinant nucleic acid comprising a second expressible nucleotide sequence configured to modulate the expression and/or function of said first nucleotide sequence, and
- non-comprised by its genome a nucleic acid comprising a third nucleotide sequence configured to inhibit the expression and/or function of said second nucleotide sequence.

2. The nucleic acid modified biological cell, wherein said second expressible nucleotide sequence is configured to inhibit the expression of said first nucleotide sequence.

3. The nucleic acid modified biological cell of claim 1 or 2, wherein said second expressible nucleotide sequence is integrated into a chromosome of said biological cell, and/or said second expressible nucleotide sequence is integrated into an episome of said biological cell.

4. The nucleic acid modified biological cell of any of the preceding claims, wherein said nucleic acid comprising the third nucleotide sequence is an oligonucleotide.

5. The nucleic acid modified biological cell of any of the preceding claims, wherein said second expressible nucleotide sequence and said third nucleotide sequence encode a regulatory RNA.

6. The nucleic acid modified biological cell of any of the preceding claims, wherein said second expressible nucleotide sequence encode an RNA selected from the group consisting of: shRNA (small hairpin RNA), snRNA (small nuclear RNA), miRNA (microRNA), miRNA sponge, piRNA, IncRNA (long non-coding RNA), guide RNA, mRNA (messenger RNA), antisense transcript, transposon.

7. The nucleic acid modified biological cell of any of the preceding claims, wherein said third nucleotide sequence encode an RNA selected from the group consisting of: siRNA (small interfering RNA), ASO (antisense oligonucleotide), miRNA (microRNA), AMO (anti-miRNA), aptamer.

8. The nucleic acid modified biological cell of any of the preceding claims, wherein said biological cell is an immune cell, preferably said immune cell is selected from the group consisting of: T cell, CAR T cell, tumor infiltrating lymphocyte, donor lymphocyte infusion, NK cell, CAR NK cell, B cell, dendritic cell, macrophage, tumor infiltrating macrophage, hematopoietic stem cell, including allogenic afore-listed cells.

9. The nucleic acid modified biological cell of any of the preceding claims, wherein said target encoded by the first expressible nucleotide sequence is a protein.

10. The nucleic acid modified biological cell of any of the preceding claims, wherein said first expressible nucleotide sequence encoding the target is a gene selected from any of the two groups consisting of:
- BRD4, p300, TET2, DNMT3, miR-15/16, miR-150, GLUT1, TOX, Blimp-1, NR4A, BATF, IRF4, NFAT, KLRG1, EOMES, TBX2, PRDM1, GZMA, HZMB, PRF1, IFNG, PD1, TIM3, LAG3, TIGIT, CTLA4, miR-146a, miR-155, MHC-I, MHC-II, miR-17-92, CX3CR1, HLA-E, HLA-G, Siglec7/9,
and/or
- IL-1, IL-6. GM-CSF, CD40L, LAG3, CD69, TCF1, BCL-6, BMI1, FOXO1, KLF2, LEF1, TCF7, IL2RA, CD27, TNF, CCR7, SELL, CD62L, miR-143, CCR5, CCR2, let7, TGFBeta, IL-12, IL-18, IL-23, T cell receptor, miR-27a, IL-7, CCL19, CCL21, CAR constructs targeting the tumor microenvironment, IL-15, IL-7, CAR construct targeting a cancer stem cell marker.

11. The nucleic acid modified biological cell of any of the preceding claims for use in medicine, preferably for use in the treatment and/or prophylaxis of a disease selected from the group consisting of: cancer, autoimmune disease, alloimmunity (rejection or graft versus host disease), viral infection, bacterial infection.

12. A method for modulating, in a nucleic acid modified cell, the expression of a first nucleotide sequence encoding a target, comprising:
1) provision of a biological cell comprising, integrated in the genome of biological cell, a first expressible nucleotide sequence encoding a target protein,
2) integrating into the genome of said biological cell a recombinant nucleic acid comprising a second expressible nucleotide sequence configured to modulate the expression and/or function of said first nucleotide sequence,
3) introducing into said biological cell outside of its genome a nucleic acid comprising a third nucleotide sequence configured to inhibit the expression and/or function of said second nucleotide sequence.

13. The method of any of the preceding claims, wherein in step (3) said nucleic acid comprising the third nucleotide sequence is introduced into said biological cell via a method selected from the group consisting of: electroporation, lipid conjugation delivery, aptamer conjugation delivery, lipid nanoparticle delivery, viral vector delivery, antibody conjugation delivery, small molecule conjugation delivery, peptide conjugation delivery, extracellular vesicle delivery.

14. The method of any of the preceding claims, wherein in step (2) said recombinant nucleic acid comprising the second expressible nucleotide sequence is introduced into said biological cell via an integrating and/or episomal virus, thereby adding the recombinant nucleic acid as part of the genome.

15. A method for the treatment of a living being in need by administering the nucleic acid modified biological cell of any of the claims 1-11.
